# EUROPEAN PATENT APPLICATION

(11) **EP 4 749 641 A1**
(43) Date of publication of application: **27.05.2026**
(21) Application number: 25217236.6
(22) Date of filing: 20.11.2025
(51) Int. Cl.: G16H 20/40, G16H 30/40, G16H 40/63

(54) **VISUALIZATION OF AN INTERNAL PROCESS OF AN AUTOMATED OPERATION**

(30) Priority: 20.11.2024 US 202418954195
(71) Applicant: Cilag GmbH International, 6300 Zug (CH)
(72) Inventor: SHELTON IV, Frederick E., Cincinnati, 45242 (US); BRADY, John E., Cincinnati, 45242 (US); JONES, Shannon L., Cincinnati, 45242 (US); DOWNING, Laura S., Cincinnati, 45242 (US); DECK, Andrew C., Cincinnati, 45242 (US); HIRSCHFELD, Weston Staney, Cincinnati, 45242 (US); CHUNG, Scotty A., Cincinnati, 45242 (US); EVERS, Anthony J., Cincinnati, 45242 (US); DAHLING, Todd M., Cincinnati, 45242 (US); CROOKER, Aaron, Cincinnati, 45242 (US); JOHNS, Christopher, Cincinnati, 45242 (US); CLARK, Devin J., Cincinnati, 45242 (US); FISHER, Douglas W., Cincinnati, 45242 (US); JAYME, Madeleine, Cincinnati, 45242 (US)
(74) Representative: Carpmaels & Ransford LLP

(57) **Abstract**

Device and methods for visualizing internal processes of an automated operation. An example device may receive an external data stream from a source external to the surgical system. The device may derive, based at least on the external data stream, decision contextual information. The device may select a surgical option associated with a surgical instrument based on the decision context information. The device may generate a visual indication of the decision context information associated with selecting the surgical option. The device may generate a control signal associated with the surgical instrument based on the selected surgical option.

## Description

### CROSS-REFERENCE TO RELATED APPLICATIONS

This application claims the benefit of the following, the disclosures of which are incorporated herein by reference in its entirety:
- Provisional U.S. Patent Application No. 63/602,040, filed November 22, 2023,
- Provisional U.S. Patent Application No. 63/602,028, filed November 22, 2023,
- Provisional U.S. Patent Application No. 63/601,998, filed November 22, 2023,
- Provisional U.S. Patent Application No. 63/602,003, filed November 22, 2023,
- Provisional U.S. Patent Application No. 63/602,006, filed November 22, 2023,
- Provisional U.S. Patent Application No. 63/602,011, filed November 22, 2023,
- Provisional U.S. Patent Application No. 63/602,013, filed November 22, 2023,
- Provisional U.S. Patent Application No. 63/602,037, filed November 22, 2023,
- Provisional U.S. Patent Application No. 63/602,007, filed November 22, 2023,
- Provisional U.S. Patent Application No. 63/603,031, filed November 27, 2023, and
- Provisional U.S. Patent Application No. 63/603,033, filed November 27, 2023.

This application is related to the following, filed contemporaneously, the contents of each of which are incorporated by reference herein:
- U.S. Patent Application No. 18/954,186, filed November20, 2024, entitled METHOD FOR MULTI-SYSTEM INTERACTION,
- U.S. Patent Application No. 18/954,199, filed November20, 2024, entitled, VISUALIZATION OF AUTOMATED SURGICAL SYSTEM DECISIONS,
- U.S. Patent Application No. 18/954,205, filed November 20, 2024, entitled VISUALIZATION OF EFFECTS OF DEVICE PLACEMENT IN AN OPERATING ROOM,
- U.S. Patent Application No. 18/954,178, filed November 20, 2024, entitled VISUALIZATION OF EFFECTS OF DEVICE MOVEMENTS IN AN OPERATING ROOM, and
- U.S. Patent Application No. 18/954,214, filed November 20, 2024, entitled DISPLAY OF COMPLEX AND CONFLICTING INTERRELATED DATA STREAMS.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

Devices and methods for visualizing internal processes of an automated operation. An example device may include a processor configured to perform one or more actions. The device may receive an external data stream from a source external to the surgical system. The device may derive, based at least on the external data stream, decision contextual information. The device may select a surgical option associated with a surgical instrument based on the decision context information. The device may generate a visual indication of the decision context information associated with selecting the surgical option. The device may generate a control signal associated with the surgical instrument based on the selected surgical option.

The surgical option may be associated with stone removal. The external data stream may include a visualization of a patient's organ, and the processor is further configured to identify, based on the visualization of the patient's organ, a potential perimeter of a stone in the patient's organ. The decision context information may include the identified potential perimeter of the stone in the patient's organ. The visual indication of the decision context information may include a visual indication of the identified potential perimeter of the stone in the patient's organ.

The surgical option may be associated with stone removal. The external data stream may include a visualization of a patient's organ. The device may identify, based on the visualization of the patient's organ, a potential perimeter of a stone in the patient's organ. The device may select a stone removal treatment location based on the identified potential perimeter of the stone in the patient's organ. The control signal associated with the surgical instrument may be generated based on the selected stone removal treatment location. The visual indication of the decision context information may include a visual indication of the identified potential perimeter of the stone in the patient's organ.

The device may obtain a plurality of surgical options associated with the surgical instrument. The device may determine, based at least on the external data stream, respective system confidence assessments that correspond to the plurality of surgical options. The decision context information may include the respective system confidence assessments that correspond to the plurality of surgical options. The visual indication of the decision context information may include the system confidence assessment that corresponds to the selected surgical option.

The external data stream may include a visualization of a patient's organ. The device may determine, based on the external data stream and the selected surgical option, a resultant visualization of the patient's organ. The visual indication of the decision context information may include a visualization of the patient's organ pre-therapy and the resultant visualization of the patient's organ post-therapy.

The surgical option may be associated with stone removal. The external data stream may include a visualization of a patient's organ. The device may identify, based on the visualization of the patient's organ, a first potential perimeter of a stone in the patient's organ and a second potential perimeter of the stone in the patient's organ, the second potential perimeter encompassing the first potential perimeter. The device may calculate a first system confidence percentage associated with the first potential perimeter and a second system confidence percentage associated with the second potential perimeter. The decision context information may include the first system confidence percentage and the second system confidence percentage. The visual indication of the decision context information may include a visual indication of the first potential perimeter of the stone and its associated first system confidence percentage, and a visual indication of the first potential perimeter of the stone and its associated second system confidence percentage.

The surgical option is associated with stone removal. The external data stream may include a visualization of a patient's organ. The device may identify, based on the visualization of the patient's organ, a potential perimeter of a stone in the patient's organ. The device may identify a plurality of potential stone removal treatment locations. The device may determine, based on the potential perimeter of the stone in the patient's organ, respective confidence assessments that correspond to the plurality of potential stone removal treatment locations. The device may select a stone removal treatment location from the potential stone removal treatment locations based on their respective confidence assessments. The control signal associated with the surgical instrument may be generated based on the selected stone removal treatment location. The visual indication of the decision context information may include a visual indication of the potential perimeter of the stone in the patient's organ.

### BRIEF DESCRIPTION OF THE DRAWINGS

FIG. 1 is a block diagram of a computer-implemented surgical system.
FIG. 2 illustrates an example surgical system in a surgical operating room.
FIG. 3 illustrates an example surgical hub paired with various systems.
FIG. 4 illustrates an example situationally aware surgical system.
FIG. 5 illustrates an example robotic arm attached to a base.
FIG. 6 illustrates an example operating room arrangement of multiple robotic arms.
FIG. 7 illustrates an example display during gallstone identification.
FIG. 8 illustrates an example display of a sphere of uncertainty.
FIG. 9 illustrates an example combination dual endoscope with visual light and infrared (IR) visualization for subsurface visualization of stones within tissue.
FIG. 10 illustrates another example dual endoscope used in gallstone detection.

### DETAILED DESCRIPTION

A more detailed understanding may be had from the following description, given by way of example in conjunction with the accompanying drawings.

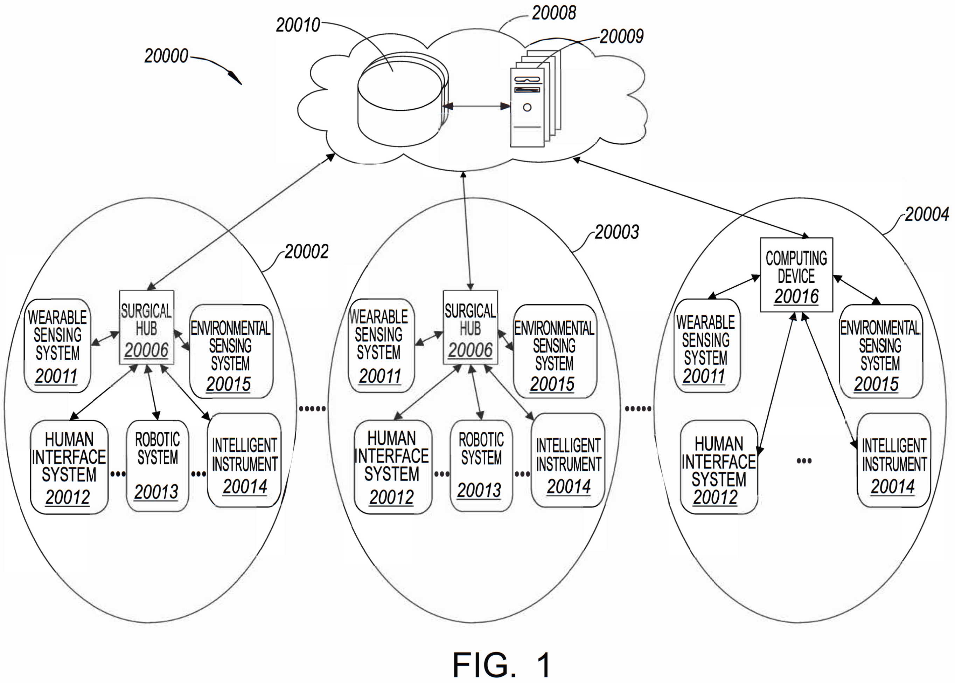

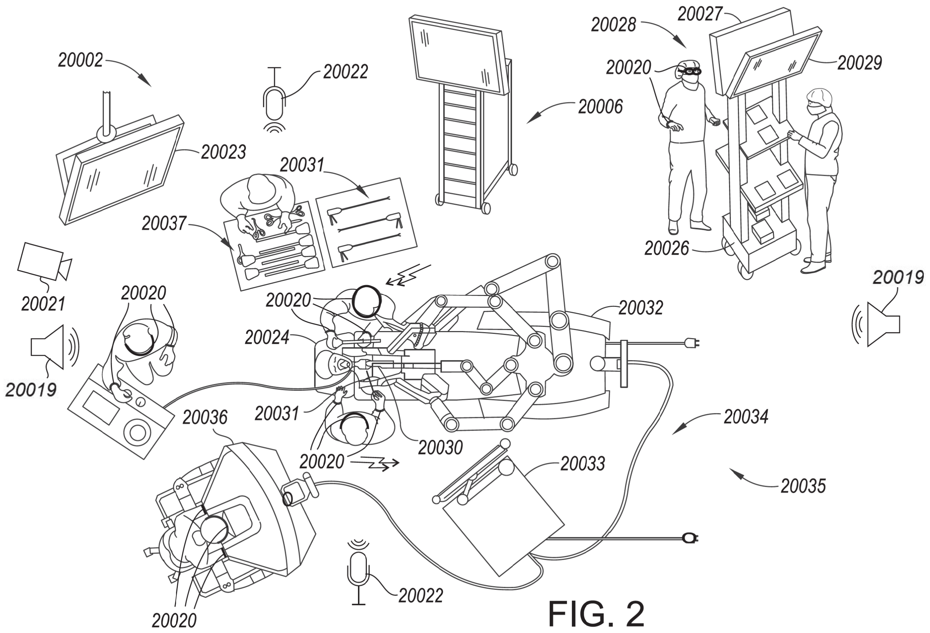

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, that is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

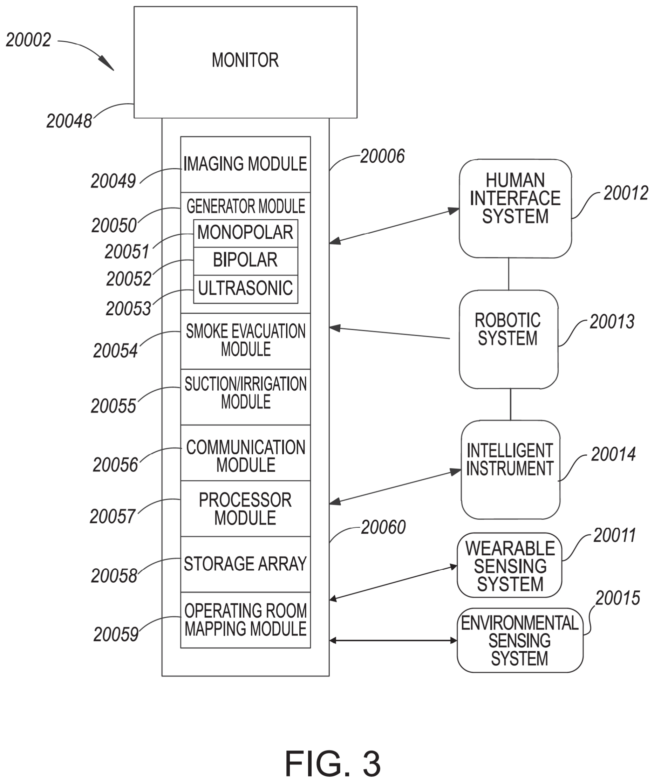

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

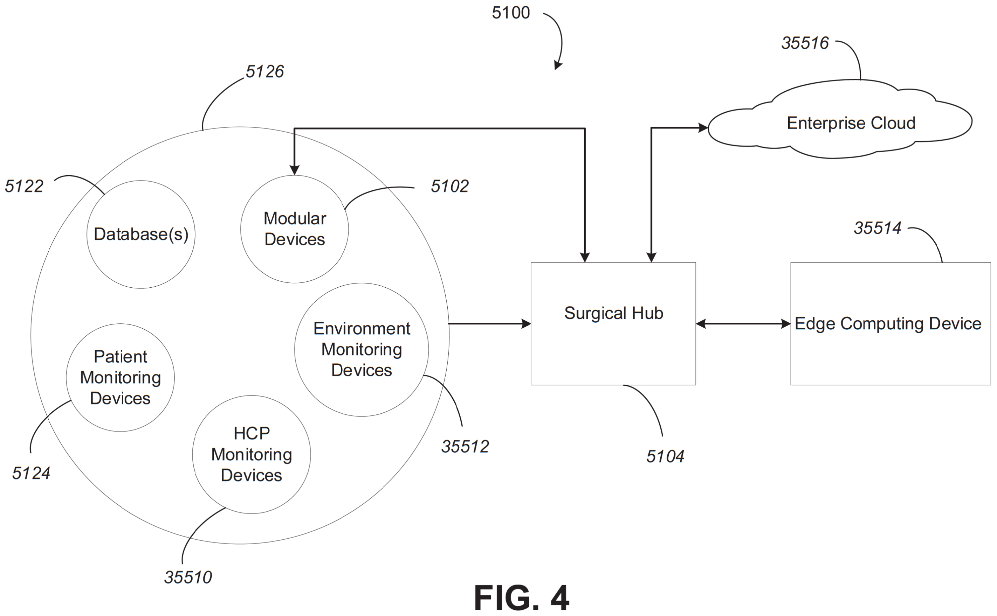

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an **RF** electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

A system may automatically make decisions during a surgical procedure. The system may display a visualization of the system-automated decision and/or information that was used to make the decision.

The system may display a visualization of an internal process of an automated operation of a smart system. The smart system may receive an external data stream from a source external to the surgical system. The system may derive decision contextual information based at least on the external data stream. The system may use the data stream from an externally supplied system to make a decision between at least two choices. The system may select a surgical option associated with a surgical instrument based on the decision context information. The system may generate a visual indication of the decision context information associated with selecting the surgical option. The system may display the decision context information or an explanation of how the decision was made (e.g., to inform the HCP of the options selected from, and why an option was selected). The context of the choice may be a perimeter or margin that the system detects. The internal process may be the probability that the system is correct (e.g., based on highlighted aspects of the image). The internal process may include therapeutic interactions associated with the decision. The system may show how a result of the decision may differ (e.g., sequentially) from the pre-operative (e.g., pre-therapy) expectations. The system may generate a control signal associated with the surgical instrument based on the selected surgical option.

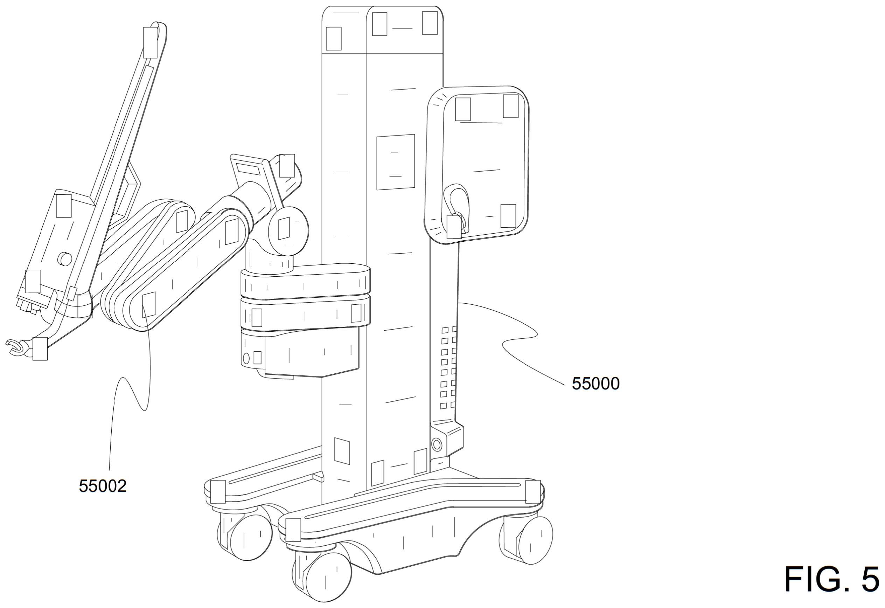

FIG. 5 illustrates an example robotic arm attached to a base 55000. In an example, the system may display (e.g., highlight or otherwise indicate) a recommended joint 55002 to move to place an end effector in the desired location. The system may further display an explanation of how the joint 55002 was selected. For example, the system may illustrate a movement path of the end effector if the joint 55002 is used. In another example, the system may indicate that the space through which the robotic arm will move if joint 55002 is used is smaller than the space the arm would move through if another joint were used.

The system may display outcomes associated with the options. This may improve the HCP's understanding of the algorithm (e.g., decision-making process). The system may demonstrate the result and aspects of the decision-making process leading to the system's decision (e.g., so that the user understands and believes the algorithm has accurately made a decision).

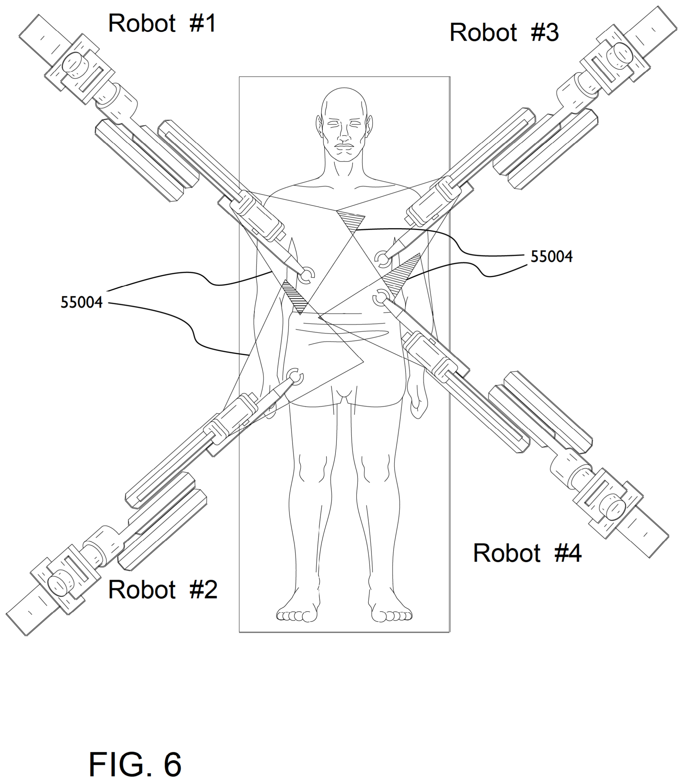

For example, FIG. 6 illustrates an operating room arrangement of multiple robotic arms. As shown, multiple robotic arms may be present in a relatively small space in an OR. The arms may have ranges of motion that overlap (e.g., as shown at 55004). In this case, the arms may collide unless adjustments are made to reduce the potential interactions. For example, one or more of the arms may not be allowed to occupy the overlapping space (e.g., at a given time). As described herein, the system may determine potential robot arm position placement and kinematic forecasting of resulting positions, movements, and/or interactions. The system may determine (and display) options for the HCP to choose from (e.g., and request that the HCP provide input). The system may output a simple, understandable display of context and choices.

In another example, the system may automatically determine which robotic arms to move (e.g., without HCP input). The system may, for example, determine that movement of robotic arm #3 will cause a collision with robotic arm #4. In this case, the system may prevent or limit movement of robotic arm #3. If the system prevents or limits movement of a robotic arm, the system may display the reason(s) for the restriction. For example, as illustrated in FIG. 6, the system may highlight or otherwise indicate the area 55004 in which the robotic arms would collide if movement were not restricted.

The system may obtain a plurality of surgical options associated with the surgical instrument. The system may determine (e.g., based at least on the external data stream) respective system confidence assessments that correspond to the plurality of surgical options. The decision context information may include the respective system confidence assessments that correspond to the plurality of surgical options. The visual indication of the decision context information may include the system confidence assessment that corresponds to the selected surgical option.

The system may indicate a confidence level that the decision made will lead to a certain result. For example, the system may indicate a confidence level of accurately identifying a gallstone or node. The system may display a confidence percentage. The system may display visual cue(s) of the effects of a decision and the confidence level. The system may determine (e.g., based on the external data stream and the selected surgical option) a resultant visualization of the patient's organ. The visual indication of the decision context information may include a visualization of the patient's organ pre-therapy and the resultant visualization of the patient's organ post-therapy.

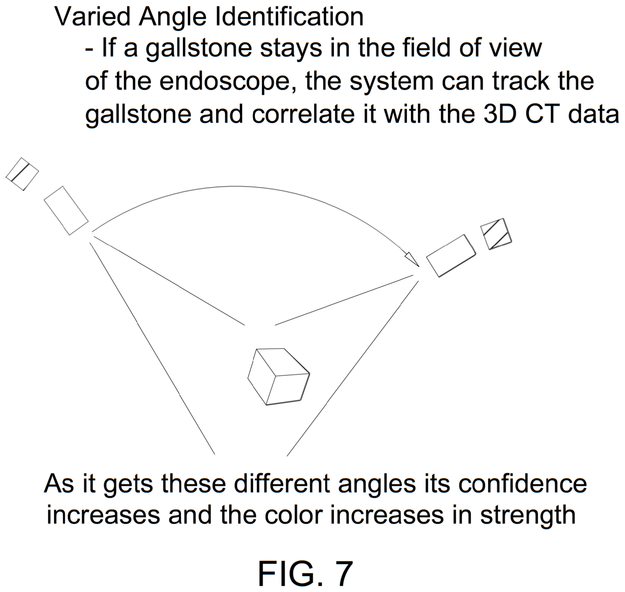

The surgical option may be associated with stone removal. The external data stream may include a visualization of a patient's organ. For example, FIG. 7 illustrates an example display during gallstone identification. As illustrated, the gallstone may be in the field of view of an endoscope. The system may track the gallstone and correlate its location with pre-operative data (e.g., from a three-dimensional CT scan). The endoscope may move around the gallstone to capture views of the gallstone from different angles. As the endoscope obtains the different views, the display may change to reflect the system's confidence in gallstone identification. For example, the color of the gallstone may change or increase in strength/brightness.

The system may identify a potential perimeter of a stone in the patient's organ based on the visualization of the patient's organ. The decision context information may include the identified potential perimeter of the stone in the patient's organ. The visual indication of the decision context information may include a visual indication of the identified potential perimeter of the stone in the patient's organ. For example, the display may have a primary indicator of a location affected by the decision and a secondary indicator of uncertainty (e.g., a potential margin of error). Error bands may vary (e.g., in both positive and negative directions) from event to event. In an example, the width of the indication may be directly correlated to the corresponding error.

The system may identify (e.g., based on the visualization of the patient's organ) a first potential perimeter of a stone in the patient's organ and a second potential perimeter of the stone in the patient's organ. The second potential perimeter may encompass the first potential perimeter. The system may calculate a first system confidence percentage associated with the first potential perimeter and a second system confidence percentage associated with the second potential perimeter. The decision context information may indicate the first system confidence percentage and the second system confidence percentage. The visual indication of the decision context information may include a visual indication of the first potential perimeter of the stone and its associated first system confidence percentage, and a visual indication of the first potential perimeter of the stone and its associated second system confidence percentage.

The system may offset indications on display to mitigate risk correlated to error. The system may have latency in communication, mechanical aspects, and/or measurement uncertainty. For example, as the system works faster, the likelihood of error in what is displayed increases. The system may intentionally offset the data display to combat the increased chance of error. A positional indicator may be shifted to the high end to represent the highest risk possibility to the user and/or patient.

The system may use previously determined data in the confidence prediction. The system may have intrinsic uncertainty when introduced into a new environment and/or operation. As subsequent actions and/or firings are performed by the system, the system may incorporate that data into its confidence predictions. This may cause the system to be more (or less) confident in future decisions.

The system may indicate a percent confidence related to timing.

A secondary data source may be used to build confidence in a primary data source. For example, an endocutter knife may be visualized as it moves from a starting position to the channel. The knife may disappear from view while it transects tissue (e.g., an anvil).

A visual appearance of the display may change based on the confidence level. For example, intensity of visual highlighting may correlate to a higher confidence level. The intensity may be displayed as stepped intensity or a range of intensities.

Visual information (e.g., critical structure detection) may be overlaid onto an augmented reality (AR) display. The information may use fill patterns, weighting, dashes, line segments around a detected object, shape and/or color of a detected object, etc. For example, if a potential object has been identified, a square box may be placed around the object on the display. As confidence in the identification grows, the square box may transform into a fitted shape around the object.

An object that has been identified may be flagged with a yellow box (e.g., indicating caution because it is an unknown object). Once the object has been identified by the system, the object may be flagged with a fitted structure or outline around the object. The object may be flagged as red (e.g., due to its surgical criticality, for example, a critical structure, tumor, etc.).

A sensitivity setting may be adjusted to change how confident the system should be before making and/or displaying a decision. The sensitivity setting may be set by a user. The sensitivity setting may be based on market research.

If multiple objects (e.g., stones, nodes) that have different confidence levels are displayed, the system may indicate the difference by using a visual (e.g., color-based) indicator, shading with different colors, transparency/opaqueness, etc. For example, a partial circumference of stone may be outlined to depict confidence in the stone's size and shape. The system may use different color rings based on the data inputs used. The indication may be a text indicator (e.g., a numerical confidence percentage). The system may display a number the stones detected and a legend. The system may give the user the option to select a stone. For example, the user may be able to hover over (or press and hold) an object (e.g., with a mouse or eye tracking) to display additional information about that object. The information may include a size of the object, a percent confidence in object identification, a likelihood of an event associated with the object (e.g., likelihood for patient to pass a stone), etc.

During pre-operative CT scanning of a patient, gallstones may be visualized (e.g., due to the density differences compared to soft tissue structures). CT systems may be able to produce digital 3D models (e.g., polygonal files, for example, .stl format). The 3D model may be funneled into the IR endoscopic computer system (e.g., via the non-proprietary .stl format). As the endoscopic camera angle changes in orientation to the object (e.g., stone) in question, the camera may capture a two-dimensional view or shape based on the reflectivity of the subsurface object (e.g., stone). The computer may line up the views of the object (e.g., aligning the real-time view with the CT data). The system may determine a level of confidence based on how well the data matches. The data may be used to notify the surgeon of confidence levels, stone depth, stone count, etc.

Interoperative visualization of subsurface gallstones may be used to ensure that none are missed during the surgical procedure. CT scanning allow for pre-operative visualization, but positions of the objects (e.g., stones) may shift between scanning and the surgical procedure. The combination of the two visualization methods may allow the surgeon to assess real time, intuitive information and mappings (e.g., with increased surgical confidence).

The identification system may utilize one or more of the following properties from the 2D image to match with the 3D object: area, maximum cross-sectional length, the concavity of the perimeter, the convexity of the perimeter, the pointed-ness of perimeter, pre-trained ML models, porosity of the stone/object, topology of the stone, uniformity of the stone surface, length of perimeter, and/or the like.

Foreign bodies may be identified (e.g., in two-dimensional intra-operative imaging) based on a three-dimensional pre-operative model. For example, the system may perform shape-based correlation of a gallstone's location using an infrared endoscope and CT system data. The system may display a computer vision recognized margin of stone (e.g., areas around expected stone center that are more or less likely to include the stone). The system may distinguish between stones and critical structures using different colors/shading/brightness. The surgeon may toggle the view selection to highlight particular structures (e.g., stones view and nodes/other structures view). The system may distinguish between stones and critical structures using identifiers (e.g., vascular identifier, etc.).

The system may indicate an unidentified object or an object identification with low confidence. In this case, the system may prompt the surgeon to identify the objects. The system may utilize the data as an input to an Al/ML feedback loop.

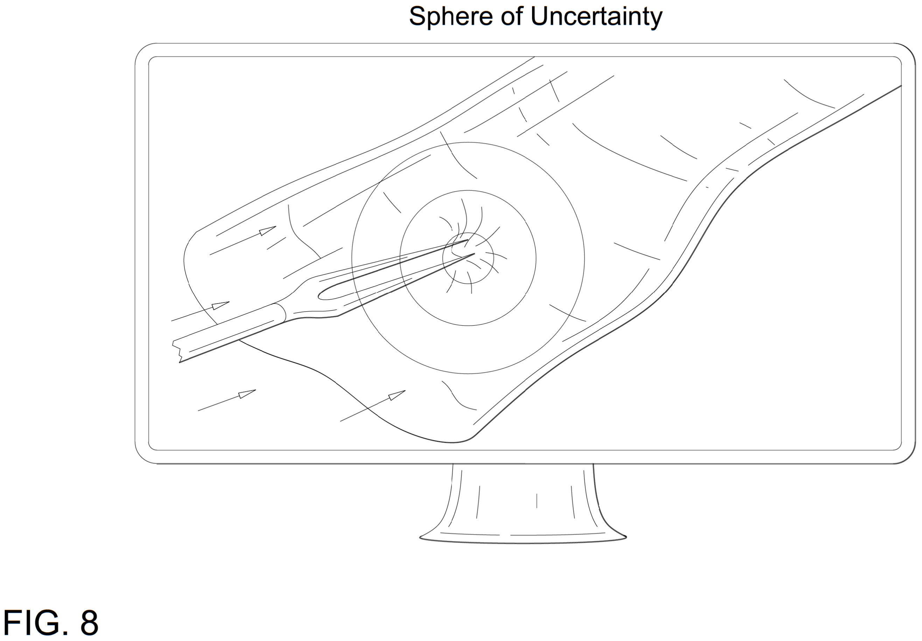

The system may display a sphere of uncertainty. FIG. 8 illustrates an example display of a sphere of uncertainty. As shown, the display may include a color-coded display of uncertainty based on the level of uncertainty or probability of being within a certain distance. For example, the area may be highlighted green if the object is within a first distance (e.g., a millimeter) of that area, highlighted yellow if the object is within a second distance (e.g., 5 millimeters) of the area, and highlighted red if the object is within a third distance (e.g., 10 millimeters) of the area. The sphere of uncertainty may adjust based on patient size, age, etc. Pre-operative CT data may be used to calibrate the sphere of uncertainty for a patient. In this way, the sphere of uncertainty may differ based on patient data.

The sphere of uncertainty may change (e.g., expand and decrease) based on the location in the body and/or device used. For example, if the object is in the lung, the sphere may be smaller than if the object is near the stomach. The sphere of uncertainty may be impacted by nearby critical structures (e.g., carotid arteries, ureter, nerves, etc.).

The sphere of uncertainty may be used to categorize and display data uncertainty (e.g., expected distance from critical structures). After identification, the system may indicate that an object is a stone or critical structure by changing the visual representation of the identified objects (e.g., based on surgical importance). For example, objects may be differentiated using color, line thickness, perimeter of the shape, type of shape (e.g., diamond, square, circle), and/or the like.

The system may change visual indications based on current surgical steps and/or actions. For example, the visual indicator may indicate the direction of removal of a surgical object (e.g., the system can indicate the direction that the kidney stones should be removed by the surgeon based on other surgical criteria). The system may indicate the next action to be taken. For example, if the system identifies multiple kidney stones, the system may identify the kidney stone that makes the most logical sense to remove first (e.g., by highlighting that stone in a distinct color).

The system may select a stone removal treatment location based on the identified potential perimeter of the stone in the patient's organ. The control signal associated with the surgical instrument may be generated based on the selected stone removal treatment location. The visual indication of the decision context information may include a visual indication of the identified potential perimeter of the stone in the patient's organ.

The system may identify (e.g., based on the visualization of the patient's organ) a potential perimeter of a stone in the patient's organ. The system may identify a plurality of potential stone removal treatment locations. The system may determine (e.g., based on the potential perimeter of the stone in the patient's organ) respective confidence assessments that correspond to the plurality of potential stone removal treatment locations. The system may select a stone removal treatment location from the potential stone removal treatment locations based on their respective confidence assessments. The control signal associated with the surgical instrument may be generated based on the selected stone removal treatment location. The visual indication of the decision context information may include a visual indication of the potential perimeter of the stone in the patient's organ.

The system may merge types of visualization. The merged visualization may depend on the overlap and/or density of visualized information. In an example, the system may initially identify several potential objects of interest (e.g., which may be flagged in yellow). The system may identify the individual objects and adjust the color of the label to indicate the objects are surgically critical objects (e.g., to highlight their importance). After the system has identified the objects, the system may group the objects with a similar type and/or nature, and/or objects co-located near one another. The system may flag the group of related objects as a single object (e.g., to reduce visual clutter for the surgeon and/or staff).

If the surgeon proceeds against instructions recommended by the system, the system may perform one or more actions. For example, the system may output surgical reminders for actions, display visual cues for the system, etc. If the surgeon is proceeding in a step or manner that is in conflict with the action suggested by the system, the system may provide visual indicators for the action. For example, the visual indicators may include direct visual cues, such as further illuminating, highlighting, or blinking an area to indicate that another action has been recommended. For example, the displayed image of the first recommended stone for removal may blink to indicate that it should be removed first.

The system may provide indicators (e.g., generalized visual cues) such as blinking the perimeter of the screen in yellow or red to indicate warnings or potential missteps. The system may provide audible or haptic cues to indicate warnings or potential missteps. If a misstep is detected, the system may prevent the surgeon from performing the action.

An AI/ML feedback loop may be used to improve system decision making. For example, if the surgeon deviates from the expected procedure, the system may report the deviation as an input to an AI/ML feedback loop. The Al/ML feedback loop may output an automated surgery report/transcript.

The system may compensate for stone and/or organ shifting between pre-operative imaging (e.g., CT) and intra-operative imaging. The system may anticipate positional shifting of the gallbladder based on patient orientation. The system may utilize pre-preoperative imaging (e.g., CT) and table positions to calculate and estimate gravitational shift. The system may use the pre-operative imaging to create a range of locations (e.g., region of interest) where stones may have shifted. The system may use stones/lymph nodes as markers to distort the pre-operative scan to match patient position.

The system may import previous surgical data to assist in organ shifting predictions. The system may predict how far stones may have moved based on fluid progression and/or natural body processes (e.g., bile movement through the bile duct at a specified rate). The system may utilize previous patient shift data to predict shifting in subsequent patients. The system may assess organ movement of a patient during positioning to determine approximate organ shifting (e.g., using external markers).

The system may indicate if it is unsure if an object is a stone or node. The system may indicate if surgeon input is needed. The system may request surgeon input via a prompt or flashing icon on a screen or AR overlay. The system may adjust the transparency, color, brightness, etc. of objects to show the identification confidence level. For example, non-identified stones (low confidence, or unsure) may appear as a different color than identified stones and/or may appear with a prompt for surgeon input.

The system may display stone margins (e.g., perimeters) that show the center of stone at a higher confidence and show less confidence as the margin is expanded outside the stone (e.g., in a bulls-eye manner). The system may update stone identifier parameters to account for a give case (e.g., based on surgeon inputs). The system may prompt the surgeon that the parameters have been updated. The system may ask the surgeon whether the system should proceed with auto-identification of stones. The system may output an on-screen display of the total quantity of identified masses. For example, the display may indicate auto-identified stones (e.g., "Stones auto-identified: 6"), auto-identified lymph nodes (e.g., "Lymph nodes auto-identified: 4"), and/or unidentified objects (e.g., "Items pending review: 3").

If the system identifies an object as a stone and the surgeon determines the object was incorrectly identified, the system may display that a surgeon override is detected. The system may ask the surgeon whether the system should report the identified object to a stone identification database. If the surgeon selects yes, the system may send information to the AI/ML cloud. If the surgeon selects no, the system may request whether to include the override in a surgery output report. The system may request that the surgeon indicate a reason for the override. The prompt may be presented to a circulating nurse to allow the surgeon to remain focused on the task at hand. The circulating nurse may record the reason for deviation from system recommendations.

If a stone is removed, the system may turn off the color/transparency indicator for that stone (or change to reflect the surgeon assessment). The system may determine if a non-gallstone was removed. The surgeon may confirm or deny whether the removed item was a stone. The system may request data to determine whether the item removed was a stone.

An in-surgery identification tool may be used to obtain stone identification feedback (e.g., prior to or after removal). For example, the identification tool may use one or more metrics (e.g., hardness, pH, density, tissue impedance, RF device impedance, harmonic tissue detection, etc.).

The system may identify if a gallstone has not (but should be) been removed. The system may overlay a gallstone identification chart from pre-operative and intra-operative imaging. The display may be updated in real time (e.g., change color/transparency after removal in the overlay). For example, after removal, the system may place an x over the stone or change transparency/color on the overlay. The system may use a generative fill (e.g., remove the stone from the pre-operative image to give the surgeon visual input that the stone has been removed). The surgeon may be able to toggle between before/after removal images. If the surgeon progresses past the stone removal, the system may indicate any stones have not been addressed.

At the completion of the surgery, the (e.g., all) stones may be assigned a treatment method (e.g., surgical removal, left to pass naturally, left due to access limitations, ultrasonic emulsification, left because the object was not correctly identified as a stone, etc. A data summary table may record the number of gallstones, the treatment method, relevant metrics, and/or linked pathology.

A smart data collection system may be used for foreign body removal tracking. A volumetric estimate of the foreign bodies may be determined based on pre-operative scanning. Removed foreign bodies may be placed on a tray for weighing (e.g., if typical density of body is known) or into fluid to measure volume. The measurement may be used to compare the volume of foreign body relative to the estimated total volume needed to be removed. If the removed volume is less than intended, the system may use this information to identify fractures of the stones.

A surgeon may gain confidence in the system's ability to make decisions. The system's decision-making approach may differ based on the user/surgeon, whether a device is new or existing, whether a user is new or existing, etc. The display may change as the user changes. Display preferences may follow users. The display may change based on a machine change (e.g., based on data), a number of times the machine has displayed information, surgeon performance; number of steps performed correctly, time between steps, eye tracking (e.g., surgeon not looking at information, machine offers to stop), surgeon input to alter the display, and/or the like.

Machine history may be used to determine an amount of information that the surgeon wants to see. User profiles may be used to set and save settings based on the surgery being performed. Stone identification threshold/sensitivity ratings may change based on the user. A level of detail customized for the user. Surgeon biometrics/preferences may be used in a simulation. For example, the system may store a glove size, establish an OR set-up based on user preference (e.g., OR table height, accompanying positions, etc.), settings for first time users, interns, residents, etc., and/or profiles for the surgery type (e.g., independent of the user).

Based on positional data, the surgeon may be interested in recommendations of angles of approach. The system may use data from other devices to determine angles of approach. For example, the system may use pre-operative imaging, target identification of surgical anatomy, critical structures to avoid, vessels during trocar placement, patient history, surgeon human factors data (e.g., dominant hand, height, vertical position of surgeon shoulders, etc.), patient height, trocar fulcrum, patient factors, table height, surgical position, BMI of the patient, surgical history, adhesions, OR personnel positioning, procedural approach, OR camera data, OR set-up, and/or the like.

The system may display recommendations to the surgeon. For example, the system may display a patient overlay via AR, selection options based on OR tasks prioritization, etc. The surgical planning system may allow a user to populate automated notifications or reminders to occur when a stone is encountered. The system may be used for teaching or educational purposes (e.g., surgical flow, key moment reminders, procedure checklist reminder, etc.).

The system may notify the surgeon if a stone is encountered. This may allow the surgeon to assess the stone prediction accuracy in real time (e.g., as each stone is approached in surgery). This may allow the surgeon to gain confidence in the identification system as an assistant. The system may learn from the surgeon's surgical decisions to improve future identification.

Pre-operative planning and/or imaging may be used by an automated intraoperative notification system. The system may identify and display a stone and its perimeter. If this is the first stone identification in this surgical procedure, the system may apply an ML filter to identify further stones. In another example, for cancer treatment, the system may identify tumors and their sizes. The system may explain the rationale behind identifying an object (e.g., similar size, density, etc.).

The system may read a radiograph to provide a supplemental analysis. For example, the system may identify artifacts created due to a CT machine position. The system may suggest turning the CT machine manually to remove shadow artifacts.

The system may display updates of progress within the procedure. The active information adjustment may indicate that the system is responding to real time behavior. A progress bar or other form of indicator may be displayed onto the screen. The system may be manually or automatically updated with the current procedure tasks or steps. Procedures may have unique listed items like the number of stones or stone pieces left to remove. If a stone fracture is detected, the system may automatically detect the number of pieces created. The indicator system may automatically update the display to indicate the number of pieces created. If the system monitors removal of foreign bodies, the system may automatically update the indicator to reflect the current progress into the procedure. The system may use the tool selection or tool activation patterns to detect the phase of the procedure (e.g., which may be reflected in the indicator system).

The system may display an on-screen procedure status or progression bar. The system may display a consensus of concurrent displays of the same assessment from differing points-of-view or customized for different HCPs. The consensus may be displayed relative to common landmarks or features of the image. These linked or cooperatives areas may be displayed with a common color, shape, digital badge, and/or notation. This may allow the HCP to switch views of the common display and re-orient themselves relative to the new-point-of-view. The images may be alternated to give the user a clear understanding of the different points-of-view and the reference shift.

Consensus references may enable an HCP to move from one view to another and re-orient themselves. For example, the consensus references may include tags or markings fixed in space to create a common reference point, fiducial markings of equipment to indicate orientation and location, and/or a common reference point for multiple images.

HCPs may be better able to re-orient themselves during a procedure due to the ability to change viewpoints. For example, a smart circular stapler (e.g., with an integrated camera) may be inserted into the patient. The staff may shift between the transanal view of the stapler during insertion and a laparoscopic camera view. The staff may be able to re-orient themselves to the procedure because they can quickly switch between different views.

The system may display images related to the movement of the system. The display may show a picture-in-picture image. The user may be able to perform a remote override of what is present on the screens. If multiple displays show different images, notification may display differently on the different screens or may be the same on both screens. Depending on users present, an interest in notifications may change.

Different HCPs may prefer that some data be displayed instead of other data. For example, a radiologist may monitor a cone-beam CT (for identification of tumor margins, instrument location, instrument orientation, critical structure identification, etc.), and the surgeon using an endoscope laparoscope may prefer that the display show data associated with the scope (e.g., to improve local control of the instrument).

HCPs may monitor the same region for different surgical reasons. For example, robotic imaging operated by the robotic control surgeon, and a laparoscope or endoscope being controlled at the table by the surgical assistant or second surgeon may both look at the same local area with differing visualization systems and purposes.

Multiple displays may show different aspects of the same data feed for different HCPs. For example, a back table nurse may look at the surgical procedure flow with emphasis on product usage and stock, and a surgeon may look at a related view of the surgical plan by looking at the instrument functional operation.

Multiple imaging arrays may be used to monitor different perspectives of a coincident location for different aspects of the surgical field and/or view. For example, the system may identify a stone size and bile duct size from pre-operative imaging (e.g., CT) and intra-operative imaging (e.g., EBUS). The system may use both datasets for display and decision making to determine whether a stone is removed or allowed to pass naturally, where the stone should be accessed from (e.g., via duct, or otherwise), etc. The surgeon/OR team may review the EBUS and CT data separately intraoperatively. The data may be presented to different users (e.g., CN sees CT, surgeon sees EBUS), or at different times (e.g., surgeon compares EBUS to CT by opening CT scan data in a minimized image).

Pre-operative and/or intra-operative imaging may be used to determine the cross-sectional area of a bile duct and/or stone size, compare the sizes to determine whether the stone may get stuck in the duct. The system may inform the surgeon of the stone size relative to the duct size (e.g., intraoperatively during interrogation of stone with EBUS). The system may display the size of stone as a percentage of the duct size (e.g., after surgeon uses EBUS on a stone).

The system may indicate a gallstone pass rate confidence using multiple data streams. The system may use a multi-spectral view to differentiate between differing characteristics of tissue. For example, multiple data streams in different light frequencies may be used to make different assessments regarding the same tissue. The data streams may be displayed differently to the OR team (e.g., Surgeon A may see IR data showing deep tissue penetration to ID stones, Surgeon B may see visual light while performing dissection to reach said stones).

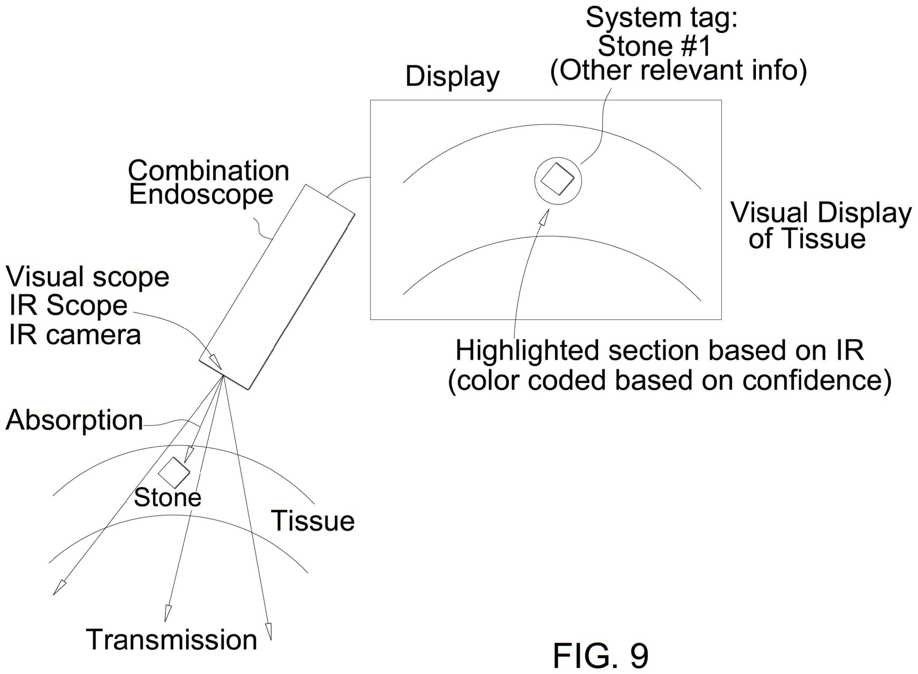

As shown in FIG. 9, a combination dual endoscope may use visual light and infrared (IR) visualization for subsurface visualization of stones within tissue (e.g., IR in the 600-1600 nanometer range may penetrate up to 8-10mm of soft tissue but not the gallstones themselves). The scope may be used to identify potential stones within the tissue, allowing the surgeon real time feedback on the stone's location. Technology may be combined with other visualization/scanning techniques (e.g., CT, ultrasound, etc.) to increase confidence levels. Data may be sent to a user interface where a visual endoscopic image is displayed with an overlay of the infrared image, as shown in FIG. 9. The color and/or brightness of the detected stone may change based on the system's confidence in correctly identifying the stone. Depending on the IR absorption or transmission in the field of view, the overlay may display a map/information regarding stone location (e.g., with potential locations highlighted based on the system confidence of stone presence).

Users may have custom displays of data (e.g., from an endoscopic multi-band IR camera, an endoscopic IR camera, a data guidance display, etc.). In stone identification, the system may use a fluorescing, radio opaque, or other imaging detector to improve the contrasting of the difference between to the two indistinguishable options.

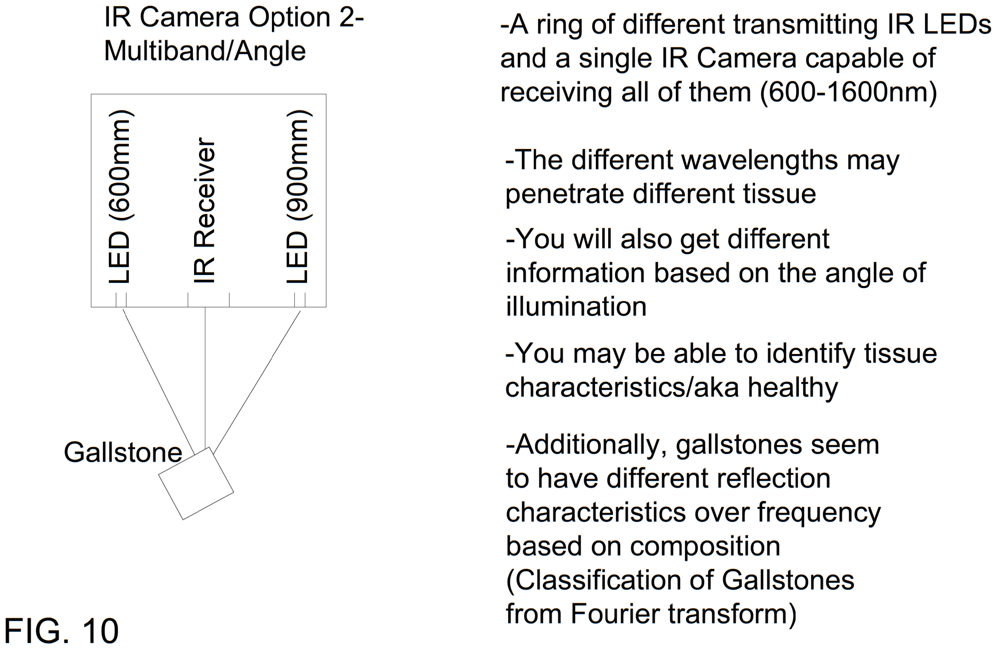

FIG. 10 illustrates another example dual endoscope used in gallstone detection. As shown, the endoscope may have 600 and 900 nanometer wavelength IR LEDs, and an IR receiver. The IR receiver may be capable of receiving the wavelengths of both IR LEDs. The different wavelengths may penetrate different tissues and/or depths. The IR receiver may receive varying information based on the angle of illumination of an area. For example, the endoscope may use the information to identify tissue characteristics (e.g., health, thickness, etc.). The system may use the different wavelengths and angles of illumination to identify foreign bodies, such as gallstones. Gallstones may have different reflection characteristics when illuminated with different frequencies. The reflection characteristics may change based on gallstone composition. The system may use an algorithm to classify objects as gallstones.

To aid in differentiating stones from nodes, the system may use a dye that would react to either the stone or node (e.g., but not the other). Such dye may create additional contrast in pre-operative imaging. The system may classify the likelihood that an object is a stone or node based on statistical size/shape/location of the object (e.g., IE nodes are on average smaller than X dimension, so a larger item is more likely to be a stone).

Foreign body identification may be used in gallstone removal. The system may display an uncertainty of portions of the overall decision. The display may include context to show aspects of which the system is fairly certain and aspects with a higher probability of misidentification (e.g., due to the subjective nature or erroneous implications).

For example, a multi-spectral imaging system with IR and fluorescing capabilities may be used to identify underlying critical structures (e.g., in order to minimize inadvertent collateral damage during dissection). Structures buried within surrounding tissues may obscure the crisp outlines of the critical structure. As the surgeon removes more surrounding tissues, the line defining the edge of the structure may become more defined or thinner.

The zone of the margin on the structure may be estimated using a combination of thermal gradients (e.g., to estimate how deep the structure is buried) and one or more other multi-spectral wavelengths to create a "zone of uncertainty." The system may communicate the zone of uncertainty to the surgeon. The zone of uncertainty may be defined between the most conservative and most aggressive possibilities of the determined edges of the structure. As the system becomes more certain of the edge location, the zone may change colors, become more defined, or decrease in size (e.g., until specifically fixated around the edge of the structure).

For example, the system may detect a zone of uncertainty around cancer that is being resected. The tumor may have a definitive and/or different structural configuration compared to the surrounding healthy tissues. The zone may be defined as the amount/area of tissue to be removed so that no cancerous tissue is left behind. One or more imaging techniques may be used for intraoperative margin assessments. For example, imaging techniques may include computed tomography (e.g., cone mean CT), elastic scattering spectroscopy (ESS), optical coherence tomography (OCT), tagged florescence, and/or the like. The imaging techniques may have benefits and shortcomings (compared to other techniques) to assess the margin (e.g., until histologic sectioning is done post transection). Inadequate margins may create significant issues in cancer treatment. For example, the occurrence of inadequate margins is roughly 5% in lung cancer treatments, 15-20% in breast, prostate, and colon cancer treatments, and up to 40-60% in GYN cancer treatments. Margin may be a portion of the equation to be solved. The surgeon may balance retention of remaining healthy organ volume, proximity to critical structures, access concerns, dealing with related anatomic structures, and/or the like. These tradeoffs may influence the size of the margin used. With algorithmic interpretation of the imaging and ML and Al assessments of previous surgeries, aspects, and context relative to the current situation, the system may determine a zone of uncertainty on the edge of the impacted tissue and the completely healthy unaffected tissues.

The system may display multiple zones of uncertainty overlayed over each other. Such an overlay may enable the surgeon to choose the zone of uncertainty that is more important, more likely to be incorrect, or more likely to result in the best outcome for the patient. The system may overlay probability average lines overlaid on the zones of uncertainty to show the mean, median, or most likely guess of where the margins end.

If zones overlap in a manner that makes it improbable that there is a delineated line between the zones which can be used for the surgical step, the system or surgeon may select one of the zones to use. In this case, the overlapping zone may be highlighted to request that the surgeon to select a zone (e.g., define which zone is more critical in this situation, more trustworthy, etc.).

Example 1. A surgical system comprising:
a processor configured to:
receive an external data stream from a source external to the surgical system;
derive, based at least on the external data stream, decision contextual information;
select a surgical option associated with a surgical instrument based on the decision context information;
generate a visual indication of the decision context information associated with selecting the surgical option; and
generate a control signal associated with the surgical instrument based on the selected surgical option.

Example 2. The surgical system of example 1, wherein the surgical option is associated with stone removal, and the external data stream comprises a visualization of a patient's organ, and the processor is further configured to:
identify, based on the visualization of the patient's organ, a potential perimeter of a stone in the patient's organ, wherein the decision context information comprises the identified potential perimeter of the stone in the patient's organ, and the visual indication of the decision context information comprises a visual indication of the identified potential perimeter of the stone in the patient's organ.

Example 3. The surgical system of example 1 or 2, wherein the surgical option is associated with stone removal, and the external data stream comprises a visualization of a patient's organ, and the processor is further configured to:
identify, based on the visualization of the patient's organ, a potential perimeter of a stone in the patient's organ; and
select a stone removal treatment location based on the identified potential perimeter of the stone in the patient's organ, wherein the control signal associated with the surgical instrument is generated based on the selected stone removal treatment location, and the visual indication of the decision context information comprises a visual indication of the identified potential perimeter of the stone in the patient's organ.

Example 4. The surgical system of any one of examples 1-3, wherein the processor is further configured to:
obtain a plurality of surgical options associated with the surgical instrument; and
determine, based at least on the external data stream, respective system confidence assessments that correspond to the plurality of surgical options, wherein the decision context information comprises the respective system confidence assessments that correspond to the plurality of surgical options, and the visual indication of the decision context information comprises the system confidence assessment that corresponds to the selected surgical option.

Example 5. The surgical system of any one of examples 1-4, wherein the external data stream comprises a visualization of a patient's organ, and the processor is further configured to:
determine, based on the external data stream and the selected surgical option, a resultant visualization of the patient's organ, wherein the visual indication of the decision context information comprises a visualization of the patient's organ pre-therapy and the resultant visualization of the patient's organ post-therapy.

Example 6. The surgical system of any one of examples 1-5, wherein the surgical option is associated with stone removal, and the external data stream comprises a visualization of a patient's organ, and the processor is further configured to:
identify, based on the visualization of the patient's organ, a first potential perimeter of a stone in the patient's organ and a second potential perimeter of the stone in the patient's organ, the second potential perimeter encompassing the first potential perimeter; and
calculate a first system confidence percentage associated with the first potential perimeter and a second system confidence percentage associated with the second potential perimeter, wherein the decision context information comprises the first system confidence percentage and the second system confidence percentage, and wherein the visual indication of the decision context information comprises a visual indication of the first potential perimeter of the stone and its associated first system confidence percentage, and a visual indication of the first potential perimeter of the stone and its associated second system confidence percentage.

Example 7. The surgical system of any one of examples 1, wherein the surgical option is associated with stone removal, and the external data stream comprises a visualization of a patient's organ, and the processor is further configured to:
identify, based on the visualization of the patient's organ, a potential perimeter of a stone in the patient's organ;
identify a plurality of potential stone removal treatment locations;
determine, based on the potential perimeter of the stone in the patient's organ, respective confidence assessments that correspond to the plurality of potential stone removal treatment locations; and
select a stone removal treatment location from the potential stone removal treatment locations based on their respective confidence assessments, wherein the control signal associated with the surgical instrument is generated based on the selected stone removal treatment location, and the visual indication of the decision context information comprises a visual indication of the potential perimeter of the stone in the patient's organ.

Example 8. A method, performed by a surgical system, the method comprising:
receiving an external data stream from a source external to the surgical system;
deriving, based at least on the external data stream, decision contextual information;
selecting a surgical option associated with a surgical instrument based on the decision context information;
generating a visual indication of the decision context information associated with selecting the surgical option; and
generating a control signal associated with the surgical instrument based on the selected surgical option.

Example 9. The method of example 8, wherein the surgical option is associated with stone removal, and the external data stream comprises a visualization of a patient's organ, and the method further comprises:
identifying, based on the visualization of the patient's organ, a potential perimeter of a stone in the patient's organ, wherein the decision context information comprises the identified potential perimeter of the stone in the patient's organ, and the visual indication of the decision context information comprises a visual indication of the identified potential perimeter of the stone in the patient's organ.

Example 10. The method of example 8 or 9, wherein the surgical option is associated with stone removal, and the external data stream comprises a visualization of a patient's organ, and the method further comprises:
identifying, based on the visualization of the patient's organ, a potential perimeter of a stone in the patient's organ; and
selecting a stone removal treatment location based on the identified potential perimeter of the stone in the patient's organ, wherein the control signal associated with the surgical instrument is generated based on the selected stone removal treatment location, and the visual indication of the decision context information comprises a visual indication of the identified potential perimeter of the stone in the patient's organ.

Example 11. The method of any one of examples 8-10, wherein the method further comprises:
obtaining a plurality of surgical options associated with the surgical instrument; and
determining, based at least on the external data stream, respective system confidence assessments that correspond to the plurality of surgical options, wherein the decision context information comprises the respective system confidence assessments that correspond to the plurality of surgical options, and the visual indication of the decision context information comprises the system confidence assessment that corresponds to the selected surgical option.

Example 12. The method of any one of examples 8-11, wherein the external data stream comprises a visualization of a patient's organ, and the method further comprises:
determining, based on the external data stream and the selected surgical option, a resultant visualization of the patient's organ, wherein the visual indication of the decision context information comprises a visualization of the patient's organ pre-therapy and the resultant visualization of the patient's organ post-therapy.

Example 13. The method of any one of examples 8-12, wherein the surgical option is associated with stone removal, and the external data stream comprises a visualization of a patient's organ, and the method further comprises:
identifying, based on the visualization of the patient's organ, a first potential perimeter of a stone in the patient's organ and a second potential perimeter of the stone in the patient's organ, the second potential perimeter encompassing the first potential perimeter; and
calculating a first system confidence percentage associated with the first potential perimeter and a second system confidence percentage associated with the second potential perimeter, wherein the decision context information comprises the first system confidence percentage and the second system confidence percentage, and wherein the visual indication of the decision context information comprises a visual indication of the first potential perimeter of the stone and its associated first system confidence percentage, and a visual indication of the first potential perimeter of the stone and its associated second system confidence percentage.

Example 14. The method of any one of examples 8-13, wherein the surgical option is associated with stone removal, and the external data stream comprises a visualization of a patient's organ, and the method further comprises:
identifying, based on the visualization of the patient's organ, a potential perimeter of a stone in the patient's organ;
identifying a plurality of potential stone removal treatment locations;
determining, based on the potential perimeter of the stone in the patient's organ, respective confidence assessments that correspond to the plurality of potential stone removal treatment locations; and
selecting a stone removal treatment location from the potential stone removal treatment locations based on their respective confidence assessments, wherein the control signal associated with the surgical instrument is generated based on the selected stone removal treatment location, and the visual indication of the decision context information comprises a visual indication of the potential perimeter of the stone in the patient's organ.

Example 15. A surgical system comprising:
a processor configured to:
receive an external data stream associated with a patient's organ;
obtain a plurality of surgical options associated with a surgical instrument;
determine, based at least on the external data stream, respective system confidence assessments that correspond to the plurality of surgical options;
select, from a plurality of surgical options, a surgical option based on their respective system confidence assessments;
generate a control signal associated with the surgical instrument based on the selected surgical option; and
generate a visual indication of the system confidence assessment that corresponds to the selected surgical option.

Example 16. The surgical system of example 15, wherein the surgical options are associated with stone removal, and the external data stream comprises a visualization of the patient's organ, and the processor is further configured to:
identify, based on the visualization of the patient's organ, a potential perimeter of a stone in the patient's organ;
identify a plurality of potential stone removal treatment locations as the plurality of surgical options;
determine, based on the potential perimeter of the stone in the patient's organ, respective confidence assessments that correspond to the plurality of potential stone removal treatment locations; and
select a stone removal treatment location from the plurality of potential stone removal treatment locations based on their respective confidence assessments, wherein the control signal associated with the surgical instrument is generated based on the selected stone removal treatment location, and the visual indication of the system confidence assessment comprises a visual indication of the potential perimeter of the stone in the patient's organ.

Example 17. The surgical system of example 15 or 16, wherein the surgical options are associated with stone removal, and the processor is further configured to:
identify, based on the external data stream, a first potential perimeter of a stone in the patient's organ and a second potential perimeter of the stone in the patient's organ, the second potential perimeter encompassing the first potential perimeter; and
calculate a first system confidence percentage associated with the first potential perimeter and a second system confidence percentage associated with the second potential perimeter, wherein the visual indication of the system confidence assessment comprises a visual indication of the first potential perimeter of the stone and its associated first system confidence percentage and a visual indication of the first potential perimeter of the stone and its associated second system confidence percentage.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random access memory (RAM), a register, cache memory, semiconductor memory devices, magnetic media such as internal hard disks and removable disks, magnetooptical media, and optical media such as CD-ROM disks, and digital versatile disks (DVDs). A processor in association with software may be used to implement a radio frequency transceiver for use in a WTRU, UE, terminal, base station, RNC, or any host computer.

## Claims

1. A surgical system comprising a processor configured to:
receive an external data stream from a source external to the surgical system;
derive, based at least on the external data stream, decision contextual information;
select a surgical option associated with a surgical instrument based on the decision context information;
generate a visual indication of the decision context information associated with selecting the surgical option; and
generate a control signal associated with the surgical instrument based on the selected surgical option.

2. The surgical system of claim 1, wherein the surgical option is associated with stone removal, and the external data stream comprises a visualization of a patient's organ, and the processor is further configured to:
identify, based on the visualization of the patient's organ, a potential perimeter of a stone in the patient's organ, wherein the decision context information comprises the identified potential perimeter of the stone in the patient's organ, and the visual indication of the decision context information comprises a visual indication of the identified potential perimeter of the stone in the patient's organ.

3. The surgical system of claim 1 or claim 2, wherein the surgical option is associated with stone removal, and the external data stream comprises a visualization of a patient's organ, and the processor is further configured to:
identify, based on the visualization of the patient's organ, a potential perimeter of a stone in the patient's organ; and
select a stone removal treatment location based on the identified potential perimeter of the stone in the patient's organ, wherein the control signal associated with the surgical instrument is generated based on the selected stone removal treatment location, and the visual indication of the decision context information comprises a visual indication of the identified potential perimeter of the stone in the patient's organ.

4. The surgical system of any one of the preceding claims, wherein the processor is further configured to:
obtain a plurality of surgical options associated with the surgical instrument; and
determine, based at least on the external data stream, respective system confidence assessments that correspond to the plurality of surgical options, wherein the decision context information comprises the respective system confidence assessments that correspond to the plurality of surgical options, and the visual indication of the decision context information comprises the system confidence assessment that corresponds to the selected surgical option.

5. The surgical system of any one of the preceding claims, wherein the external data stream comprises a visualization of a patient's organ, and the processor is further configured to:
determine, based on the external data stream and the selected surgical option, a resultant visualization of the patient's organ, wherein the visual indication of the decision context information comprises a visualization of the patient's organ pre-therapy and the resultant visualization of the patient's organ post-therapy.

6. The surgical system of any one of the preceding claims, wherein the surgical option is associated with stone removal, and the external data stream comprises a visualization of a patient's organ, and the processor is further configured to:
identify, based on the visualization of the patient's organ, a first potential perimeter of a stone in the patient's organ and a second potential perimeter of the stone in the patient's organ, the second potential perimeter encompassing the first potential perimeter; and
calculate a first system confidence percentage associated with the first potential perimeter and a second system confidence percentage associated with the second potential perimeter, wherein the decision context information comprises the first system confidence percentage and the second system confidence percentage, and wherein the visual indication of the decision context information comprises a visual indication of the first potential perimeter of the stone and its associated first system confidence percentage, and a visual indication of the first potential perimeter of the stone and its associated second system confidence percentage.

7. The surgical system of any one of the preceding claims, wherein the surgical option is associated with stone removal, and the external data stream comprises a visualization of a patient's organ, and the processor is further configured to:
identify, based on the visualization of the patient's organ, a potential perimeter of a stone in the patient's organ;
identify a plurality of potential stone removal treatment locations;
determine, based on the potential perimeter of the stone in the patient's organ, respective confidence assessments that correspond to the plurality of potential stone removal treatment locations; and
select a stone removal treatment location from the potential stone removal treatment locations based on their respective confidence assessments, wherein the control signal associated with the surgical instrument is generated based on the selected stone removal treatment location, and the visual indication of the decision context information comprises a visual indication of the potential perimeter of the stone in the patient's organ.

8. A method, performed by a surgical system, the method comprising:
receiving an external data stream from a source external to the surgical system;
deriving, based at least on the external data stream, decision contextual information;
selecting a surgical option associated with a surgical instrument based on the decision context information;
generating a visual indication of the decision context information associated with selecting the surgical option; and
generating a control signal associated with the surgical instrument based on the selected surgical option.

9. The method of claim 8, wherein the surgical option is associated with stone removal, and the external data stream comprises a visualization of a patient's organ, and the method further comprises:
identifying, based on the visualization of the patient's organ, a potential perimeter of a stone in the patient's organ, wherein the decision context information comprises the identified potential perimeter of the stone in the patient's organ, and the visual indication of the decision context information comprises a visual indication of the identified potential perimeter of the stone in the patient's organ.

10. The method of claim 8 or claim 9, wherein the method further comprises:
obtaining a plurality of surgical options associated with the surgical instrument; and
determining, based at least on the external data stream, respective system confidence assessments that correspond to the plurality of surgical options, wherein the decision context information comprises the respective system confidence assessments that correspond to the plurality of surgical options, and the visual indication of the decision context information comprises the system confidence assessment that corresponds to the selected surgical option.

11. The method of any one of claims 8 to 10, wherein the external data stream comprises a visualization of a patient's organ, and the method further comprises:
determining, based on the external data stream and the selected surgical option, a resultant visualization of the patient's organ, wherein the visual indication of the decision context information comprises a visualization of the patient's organ pre-therapy and the resultant visualization of the patient's organ post-therapy.

12. A surgical system comprising a processor configured to:
receive an external data stream associated with a patient's organ;
obtain a plurality of surgical options associated with a surgical instrument;
determine, based at least on the external data stream, respective system confidence assessments that correspond to the plurality of surgical options;
select, from a plurality of surgical options, a surgical option based on their respective system confidence assessments;
generate a control signal associated with the surgical instrument based on the selected surgical option; and
generate a visual indication of the system confidence assessment that corresponds to the selected surgical option.

13. The surgical system of claim 12, wherein the surgical options are associated with stone removal, and the external data stream comprises a visualization of the patient's organ, and the processor is further configured to:
identify, based on the visualization of the patient's organ, a potential perimeter of a stone in the patient's organ;
identify a plurality of potential stone removal treatment locations as the plurality of surgical options;
determine, based on the potential perimeter of the stone in the patient's organ, respective confidence assessments that correspond to the plurality of potential stone removal treatment locations; and
select a stone removal treatment location from the plurality of potential stone removal treatment locations based on their respective confidence assessments, wherein the control signal associated with the surgical instrument is generated based on the selected stone removal treatment location, and the visual indication of the system confidence assessment comprises a visual indication of the potential perimeter of the stone in the patient's organ.

14. The surgical system of claim 12 or claim 13, wherein the surgical options are associated with stone removal, and the processor is further configured to:
identify, based on the external data stream, a first potential perimeter of a stone in the patient's organ and a second potential perimeter of the stone in the patient's organ, the second potential perimeter encompassing the first potential perimeter; and
calculate a first system confidence percentage associated with the first potential perimeter and a second system confidence percentage associated with the second potential perimeter, wherein the visual indication of the system confidence assessment comprises a visual indication of the first potential perimeter of the stone and its associated first system confidence percentage and a visual indication of the first potential perimeter of the stone and its associated second system confidence percentage.

15. A computer-readable medium comprising instructions which, when executed by a processor, cause the processor to carry out the method of any one of claims 8 to 11.
